# EUROPEAN PATENT SPECIFICATION

(11) **EP 2 163 895 B1**
(45) Date of publication and mention of the grant of the patent: **17.08.2011**
(21) Application number: 09169631.0
(22) Date of filing: 07.09.2009
(51) Int. Cl.: G01N 33/50, G01N 33/84, C12N 5/00

(54) **A method for selecting mammal transformed cells**
Verfahren zum Auswählen von transformierten Säugertierzellen
Procédé de sélection de cellules transformées de mammifère

(30) Priority: 10.09.2008 JP 2008231694
(43) Date of publication of application: 17.03.2010
(73) Proprietor: Food and Drug Safety Center, Japan, Hadano-shi, Kanagawa 257-8523 (JP)
(72) Inventor: Sasaki, Kiyoshi, Kanagawa 257-8523 (JP)
(74) Representative: Polypatent

(56) References cited:
- MEYER A L: "IN-VITRO TRANSFORMATION ASSAYS FOR CHEMICAL CARCINOGENS" MUTATION RESEARCH, vol. 115, no. 3, 1983, pages 323-338, XP002561881 ISSN: 0027-5107
- DEICHMAN G I ET AL: "CHARACTERIZATIONS OF IN-VITRO TRANSFORMED CELLS ESSENTIAL FOR THEIR" INTERNATIONAL JOURNAL OF CANCER, vol. 37, no. 3, 1986, pages 401-410, XP002561882 ISSN: 0020-7136
- WU XIN-JIANG ET AL: "Targeting ROS: selective killing of cancer cells by a cruciferous vegetable derived pro-oxidant compound." CANCER BIOLOGY & THERAPY MAY 2007, vol. 6, no. 5, May 2007 (2007-05), pages 646-647, XP002561883 ISSN: 1555-8576
- HERDENER MARCUS ET AL: "Target cell-derived superoxide anions cause efficiency and selectivity of intercellular induction of apoptosis" FREE RADICAL BIOLOGY AND MEDICINE, vol. 29, no. 12, 15 December 2000 (2000-12-15), pages 1260-1271, XP002561884 ISSN: 0891-5849
- DATABASE BIOSIS [Online] BIOSCIENCES INFORMATION SERVICE, PHILADELPHIA, PA, US; September 2006 (2006-09), TRACHOOTHAM DUNYAPORN ET AL: "Selective killing of oncogenically transformed cells through a ROS-mediated mechanism by beta-phenylethyl isothiocyanate" XP002561885 Database accession no. PREV200600553559 & CANCER CELL, vol. 10, no. 3, September 2006 (2006-09), pages 241-252, ISSN: 1535-6108
- DATABASE EMBASE [Online] ELSEVIER SCIENCE PUBLISHERS, AMSTERDAM, NL; 1993, WEISS L ET AL: "Hydrogen peroxide in the prevention and treatment of peritoneal carcinomatosis: A feasibility study" XP002561886 Database accession no. EMB-1994022297 & REGIONAL CANCER TREATMENT 1993 DE, vol. 6, no. 2, 1993, pages 98-102, ISSN: 0935-0411

## Description

### Technical Field

The present invention relates to a method for selecting transformed mammalian cells from a cell mixture comprising transformed mammalian cells and non-transformed mammalian cells.

### Background Art

In order to examine whether or not normal mammalian cells have been transformed, namely, in order to examine whether or not normal mammalian cells are in a malignantly transformed state or in a precancerous state, a transformation experiment has been carried out. At present, in order to quantify transformed cells in this experiment system, the following 3 types of methods have been established: (1) a method of counting morphologically changed colonies (colony formation method); (2) a method of counting multilayered and morphologically changed colonies (transformed foci, or foci) found in monolayered normal cells (focus formation method); and (3) a method of counting colonies formed in a soft agar medium (soft agar colony formation method). However, these methods are disadvantageous in that, since determination of transformed cells or non-transformed cells must be carried out based on the morphology of cells, such determination is subjective and requires a long period of time. Moreover, all operations ranging from the inoculation of cells to the observation thereof must be carried out by human hand.

The transformation experiment is applied to the mechanism analysis of carcinogenesis, the screening of carcinogenic substances, determination of the degree of malignancy of tumor, quality tests for examining whether or not cancer cells are contained in tissue-engineered cell products, and the like. Accordingly, it is considered extremely useful, if transformed cells can be objectively and rapidly quantified, including, several mechanized operations,

Bhas 42 is a cell that is frequently used in transformation experiments. This cell is a clone obtained by introducing an active oncogene, v-Ha-ras, in BALB/c 3T3 cells (mouse, whole embryo), and it exhibits contact inhibition. However, when an initiator or a promoter that acts as a carcinogenic substance is added to such cells and they are then cultured, several cells lose such contact inhibition and form a transformed focus. Thus, the level of carcinogenesis is quantified by counting the number of transformed foci per well (focus formation method). Specifically, the cells are inoculated on a 6-well plate or a 96-well plate. Thereafter, in the case of a system for detecting an initiator, the cells are treated from the 1^{st} to 4^{th} days after the inoculation. In the case of a system for detecting a promoter, the cells are treated from the 4^{th} to 14^{th} days after the inoculation. Thereafter, the cells are fixed on the 21^{st} day, and they are then subjected to Giemsa staining. The stained cells are observed under a microscope, and the presence of transformed foci is determined. As a medium, DMEM/F12 + 5% fetal bovine serum is used. In the case of using a 6-well plate, the cells are cultured in 2 mL of the medium. In the case of a 96-well plate, the cells are cultured in 0.1 mL of the medium.

Several Bhas 42 transformed foci are clearly stained as a result of the Giemsa staining, and they can be clearly recognized with the naked eye. However, if such transformed foci are attempted to be quantified by image analysis using a personal computer, it is extremely difficult to carry out such quantification, not only using general image analysis software, but also using special software for analyzing immunostained cells and the like. That is to say, it is necessary that cells of interest (having color different from that of other cells) should be dispersed on the entire screen, and thus, when such cells of interest are gathered at the edge of the screen, like in the transformation experiment, the cells cannot be analyzed. Naturally, morphology change cannot be analyzed, either. Further, when a 96-well plate is used, since the area of the lateral face of a well is relatively large, as compared with the area of the bottom face thereof, the transformed foci often exist on the lateral face. In such a case, image analysis for analyzing only the bottom face incorrectly determines that there are no transformed foci.

Furthermore, when the fixed Bhas 42 cells are stained with crystal violet and are then extracted with a methanol-acetate solution, followed by the measurement of absorbance, no clear difference is observed between a well containing only normal cells and a well in which the transformed foci are found.

In the case of Bhas 42 cells on the 21^{st} day after the inoculation, it is considered that normal cells have hardly grown, and that the transformed cells have actively grown. However, when a dye whose fluorescence level is changed by metabolism (alamarBlue, etc.) or a dye whose absorbance is changed by metabolism (WST-8, etc.) is added to the cells, a clear difference is not observed.

As is apparent from the experiment using Bhas 42 cells, it has been difficult to objectively quantify transformed cells in an environment in which the transformed cells coexist with normal cells. Thus, it has been essential to observe such cells with the human naked eye under a microscope.

### Prior Art Documents

Non-Patent Document 1: LeBoeuf RA, Kerckaert KA, Aardema MJ, Isfort RJ. Use of Syrian hamster embryo and BALB/c 3T3 cell transformation for assessing the carcinogenic potential of chemicals. In The Use of Short- and Medium-Term Tests for Carcinogens and Data on Genetic Effects in Carcinogenic Hazard Evaluation. Eds. McGregor DB, Rice JM, Venitt S (1999) pp. 409-425. LARC Scientific Publications, No. 146, Lyon, France.

Non-Patent Document 2: Asada S, Sasaki K, Tanaka N, Takeda K, Hayashi M, Umeda M. Detection of initiating as well as promoting activity of chemicals by a novel cell transformation assay using v-Ha-ras-transfected BALB/c 3T3 cells (Bhas 42 cells). Mutat Res, (2005) 588:7-21.

Non-Patent Document 3: O'Hayer KM, Counter CM. A genetically defined normal human somatic cell system to study ras oncogenesis in vivo and in vitro. Methods Enzymol. (2006) 407:6377-647.

The document Meyer A. L. ("In vitro transformation assays for chemical carcinogenesis", Mutation Research, Vol. 115, No: 3, pages 323-338, 1983) is a review of transformation assays and substantiates the fact that the transformation process is highly complex and that transformation assays are highly dependent on the specific assay parameters employed. The document describes only classic transformation assays using marker relating to morphological changes.

The document Deichman et al. ("Characteristics of in Vitro transformed cells essential for their in vivo survival, selection and metastasizing activity", int. J. Cancer, Vol. 37, pages 401-409, 1986) refers to a study on the effects of hydrogen peroxide on transformed STHE cells. The document shows that resistance to H₂O₂ is positively correlated with the degree of transformation and metastatic potential of STHE cells.

### Disclosure of the Invention

It is an object of the present invention to provide a method for objectively selecting transformed mammalian cells from a state in which normal mammalian cells coexist with the transformed mammalian cells.

The present inventor has found that normal cells are specifically destroyed using a medium containing active oxygen such as hydrogen peroxide, and that only the transformed 3

cells are allowed to survive and can be selected, thereby completing the present invention.

The present invention provides an in-vitro method for selecting transformed mammalian cells, which comprises treating a cell mixture comprising transformed mammalian cells and non-transformed mammalian cells with a solution containing active oxygen, so as to allow the transformed mammalian cells to selectively survive, wherein

transformed mammalian cells include pre-transformed cells.

Preferably, the solution containing active oxygen is a solution containing hydrogen peroxide, single oxygen, superoxide anion radical, or hydroxyl radical.

Preferably, the transformed mammalian cells are pre-transformed cells.

Preferably, the transformed mammalian cells are cultured cells.

Preferably, the cultured transformed mammalian cells are Bhas 42 cells, BALB/c 3T3 cells, or human cancer cells.

Preferably, the transformed mammalian cells are cancer cells or cells that are in a precancerous state.

According to the present invention, the transformed cells can be selected from normal cells using a new marker; namely a resistance to active oxygen such a hydrogen peroxide or the like. In order to quantify the transformed cells by a general colony formation method and focus formation method, fixation, staining, and observation are required. In the case of a soft agar colony formation method, such fixation and staining are unnecessary, but observation is necessary. In contrast, the method of the present invention is a simple quantification method, in which a series of, or a part of operations consisting of fixation, staining, and observation are omitted, and thus, labor and time can be significantly reduced. In addition, in the present method, the transformed cells are quantified based on a fluorescence level or absorbance, using a measurement apparatus. Hence, the method of the present invention is an objective method involving no difference caused by different measurers. Since the present invention includes simple operations such as addition of hydrogen peroxide or dyes, it is possible to automate such operations using equipment. Moreover, in the present invention, cells resistant to active oxygen such a hydrogen peroxide or the like are isolated, so as to obtain both the transformed cells and pre-transformed cells that cannot be determined by observation, thereby enabling determination.

### Brief Description of the Drawings

Figure 1 shows the selection of Bhas 42 transformed cells using hydrogen peroxide. Figure 1A shows the cells before addition of hydrogen peroxide, and Figure 1B shows the cells treated with hydrogen peroxide (0.0015%) for 24 hours.
Figure 2 shows the schedule of a hydrogen peroxide method using Bhas 42 cells.
Figure 3 shows the influence of the concentration of hydrogen peroxide and a treatment time in wells containing Bhas 42 transformed cells. Figure 3A shows wells containing no transformed foci, and Figure 3B shows wells containing transformed foci.
Figure:4 shows a match of changes in dyes by a hydrogen peroxide method with wells containing transformed foci.
Figure 5 shows the dose dependence of MCA in a hydrogen peroxide method (alamarBlue used). The filled square indicates wells containing normal cells, and the open square indicates wells containing transformed foci.
Figure 6 shows the dose dependence of MCA in a hydrogen peroxide method (WST-8 used). The filled square indicates wells containing normal cells, and the open square indicates wells containing transformed foci.
Figure 7 shows the growth curve of Bhas pre cells.
Figure 8 shows the selection of human cancer cells using hydrogen peroxide.
Figure 9 shows the selection of Bhas 42 transformed cells by BP treatment and light irradiation.
Figure 10. shows the selection of mouse lung tumor cells using hydrogen peroxide. Figure 10A shows non-addition of hydrogen peroxide, and Figure 10B shows the cells treated with hydrogen peroxide (0.015%) for 24 hours.

### Mode for Carrying Out the Invention

The in-vitro method for selecting transformed mammalian cells of the present invention is characterized in that transformed mammalian cells are allowed to selectively survive by treating a cell mixture comprising the transformed mammalian cells and non-transformed mammalian cells with a solution containing active oxygen. The transformed mammalian cells are cells obtained from an individual mammal (*in vitro* cells). As such transformed mammalian cells, the pre-transformed cells of a mammal may also be used. Specific examples of such transformed mammalian cells include cancer cells or cells that are in a precancerous state. When the cultured cells are used as cultured transformed mammalian cells, Bhas 42 cells, BALB/c 3T3 cells, human cancer cells, or the like can be used, for example.

The cells can be transformed by the use of a chemical substance, X-ray irradiation, ultraviolet ray irradiation, the introduction of oncogene, etc. Of these, a common method involving a treatment with a chemical substance will be described as a typical example. In the case of an individual animal, a chemical substance is mixed into feed and it is then given to the animal, or it is applied onto the skin. In the case of the cultured cells, after cells have been inoculated in a medium, a chemical substance is added to the medium. The number of treatments and the treatment time are changed depending on purpose or the properties of the chemical substance. In the case of cultured cells, a single treatment may be carried out for several hours. Otherwise, treatments may be continuously carried out over several weeks, until transformed cells are observed. It is to be noted that such chemical substance is generally dissolved or suspended in water, dimethyl sulfoxide (DMSO), or the like, before-use.

The type of active oxygen contained in a solution comprising active oxygen is not particularly limited in the present invention. Examples of such active oxygen that can be used herein include hydrogen peroxide, single oxygen, superoxide anion radical, and hydroxyl radical. Of these, hydrogen peroxide is particularly preferable.

The dose of active oxygen used can be selected appropriately, depending on the type of active oxygen and the type of cells. In the case of hydrogen peroxide, it is preferably used in an dose of approximately 0.000 1 % to 0.002%.

The treatment time for treating cells with a solution containing active oxygen is riot particularly limited. It is generally from approximately 1 hour or more to 72 hours or less, and preferably from 6 hours or more to 48 hours or less.

The present invention will be more specifically described in the following examples.

### Examples

### Example 1:

The present inventor has thought that, in a state in which normal, cells coexist with transformed cells, if only the normal cells are selectively destroyed using an agent, the transformed cells can be objectively selected. Thus, the inventor has screened for such agent, using Bhas 42 cells that are cultured mammalian cells. As a result, the inventor has found that hydrogen peroxide, which produces active oxygen, destroys only normal cells.

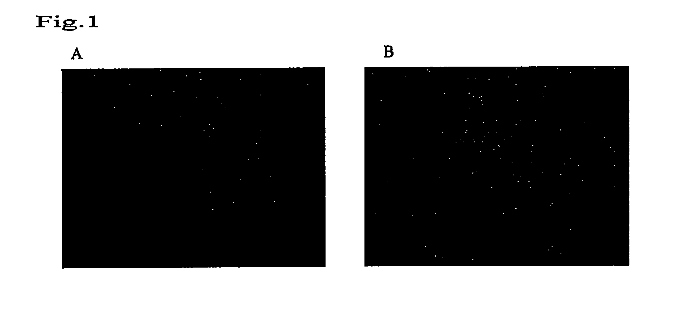

In order to observe the morphology of a transformed focus and the morphology of normal cells existing around the transformed focus before and after a treatment with hydrogen peroxide, the following experiment was carried out using 3-methylcholanthrene (MCA) as an initiator. Cells were inoculated on a 6-well plate (4000 cells/2 mL of medium/well), and on the following day, 2 µl of an MCA solution (1 mg/mL: 1,000 times higher than the final concentration that was 1 µg/mL) was added thereto, followed by a treatment for days. The appearance of transformed foci was confirmed on the 21^{st} day after the inoculation, and a certain transformed focus was then marked from the bottom of the plate. Thereafter, 0.2 mL of a medium containing hydrogen peroxide (0.0165%: 11 times higher than the final concentration that was 0.0015%) was added, and the transformed focus was then observed 24 hours after the addition of hydrogen peroxide. As a result, the morphology of the transformed focus was not changed from that before the addition of hydrogen peroxide. In contrast, cytoplasm was denatured in the normal cells (Figure 1).

### Example 2:

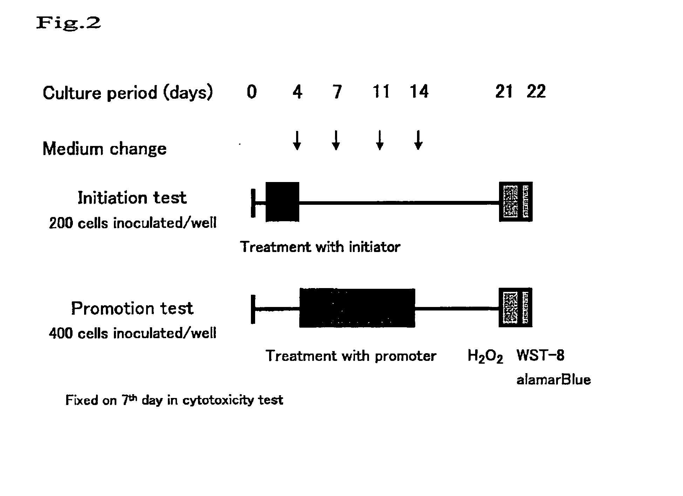

A basic experiment was carried out as follows. In a Bhas 42 cell transformation test using a 96-well plate (0.1 mL of medium/well), 0.05 mL of a medium containing hydrogen peroxide (in a concentration 3 times higher than the final concentration) was added on the 21^{st} day after cell inoculation. 1 to 24 hours later, 0.05 mL of a medium containing alamarBlue or WST-8 (0.01 mL of dye + 0.04 mL of medium) was added. Three hours after the addition of the aforementioned medium, a fluorescence level (excitation: 530 nm; measurement: 590 nm) or absorbance (450 nm) was measured (Figure 2).

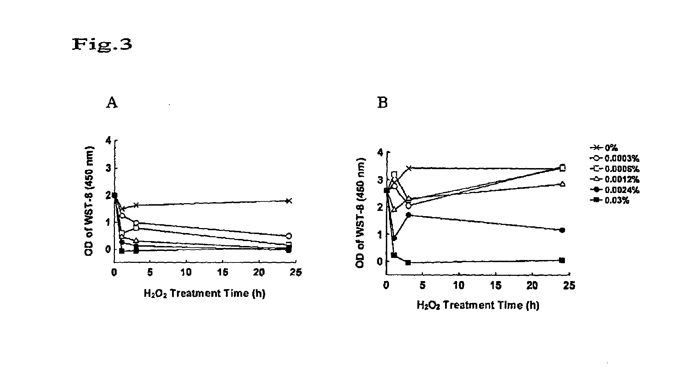

In order to find conditions under which normal cells are killed but transformed cells survive, an experiment was carried out to obtain an appropriate concentration of hydrogen peroxide and an appropriate treatment time. That is to say, a well containing only normal cells and a well containing a single transformed focus induced by the use of 1 µg/mL MCA had previously been selected, and the influence of the concentration of hydrogen peroxide and a treatment time was examined. As a result of a measurement using the absorbance of WST-8 as an indicator, it was found that hydrogen peroxide specifically destroys normal cells, depending on the concentration thereof and the treatment time therewith (Figure 3). Thus, in the Bhas 42 transformation experiment, the cells were treated with 0.0015% hydrogen peroxide, and a dye was then added to the cells 24 hours after the treatment, followed by measurement.

### Example 3:

Whether or not the transformation of Bhas 42 cells could be quantified with a dye was analyzed using MCA. The cells were inoculated (200 cells/0.05 mL of medium/well), and on the following day, 0.05 mL of a medium containing MCA (0.2, 0.6, and 2 µg/mL: all of which were 2 times higher than the final concentrations that were 0.1, 0.3, and 1 µg/mL, respectively) was added thereto. The cells were treated for 3 days, and a medium containing hydrogen peroxide (0.0045%: 3 times higher than the final concentration that was 0.0015%) was added thereto on the 21^{st} day after cell inoculation. 24 hours later, a dye was added, and measurement was then carried out. Thereafter, 0.02 mL of glutaraldehyde (2.5%: approximately 10 times higher than the final concentration that was 0.25%) was further added, and the resultant cells-were then left for-30 minutes or more for fixation. Thereafter, the cells were washed with water and were then subjected to Giemsa staining.

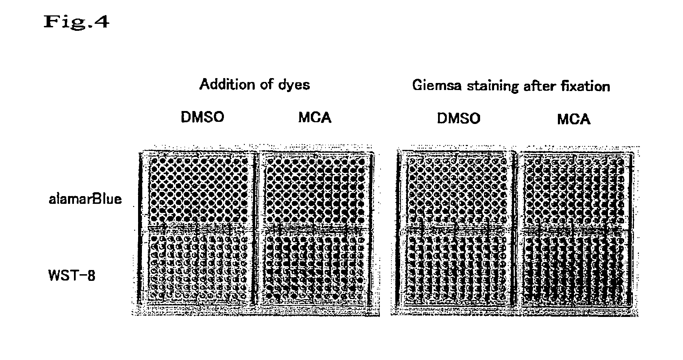

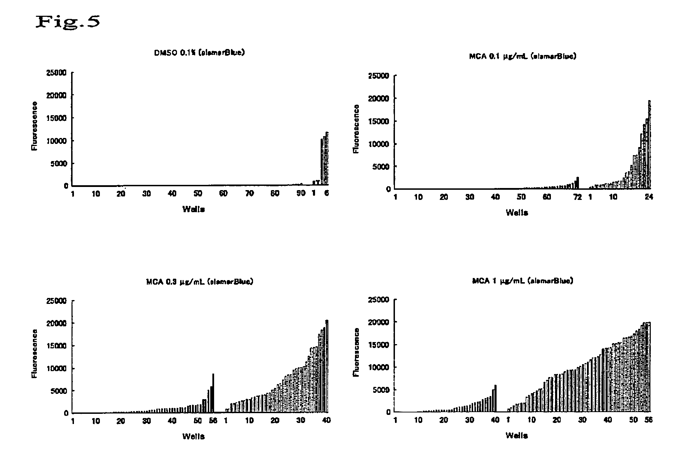

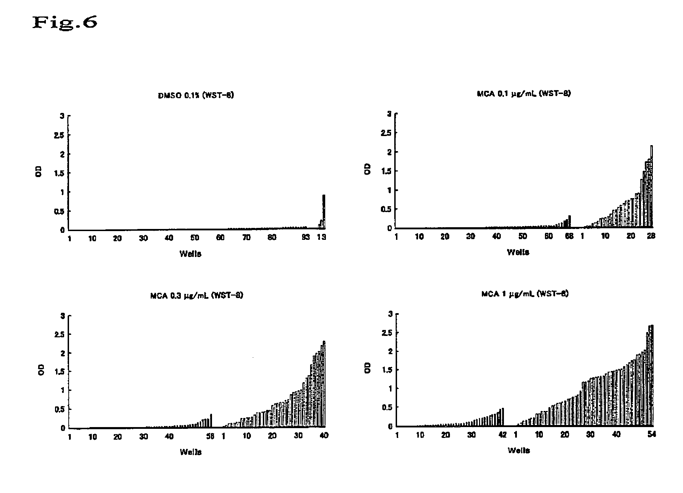

As a result, transformed foci and its fluorescence level or absorbance were increased depending on the dosage of MCA. As a result of observation of each well under a microscope, it was found that a well exhibiting a high measurement value comprised a transformed focus (Figures 4-6). The results of a method comprising adding hydrogen peroxide to cells and then quantifying the cells with a dye matched with the results of a conventional method comprising quantifying cells via observation. Thus, it was confirmed that the present invention could be applied to a transformation experiment.

Even in a case in which a transformed focus existed on the lateral face of a well, quantification could be carried out properly.

At present, automatic pipettors are available from several manufacturers. Hence, when a 96-well plate or a plate having more than 96 wells is used, simple operations such as medium dispensing can be automated. In a transformation experiment, to which the present invention is applied, hydrogen peroxide and a dye are merely added. Thus, it is possible to achieve high throughput using an automatic pipettor.

### Example 4:

There were a few well, in which no transformed foci were observed in spite of high absorbance or high fluorescence level. Thus, it was considered that Bhas 42 cells existing in such well would be cells before transformation, namely, cells in which no morphological change would occur but abnormity would be found at a molecular level. Hence, cells (Bhas preT cells; 4 strains), which had not been transformed but had not been killed by the addition of hydrogen peroxide, were isolated, and the degree of malignancy of the cells was then examined by applying a soft agar colony formation test.

In the culture of the cells on soft agar, 3 mL each of 0.5% agar medium was first dispensed into a 60-mm dish, so as to produce a base layer using such 0.5% agar medium. After the base layer had been consolidated, 2 mL of 0.33% soft agar medium containing the cells was laminated thereon, so as to prepare a cell inoculation layer (100 to 10⁴ cells/dish; the number of cells inoculated was set based on the estimated colony formation rate). After completion of the inoculation, the cells were cultured for 3 weeks. Thereafter, the number of colonies per dish was counted under a microscope.

In parallel with the soft agar culture, a conventional adhesion culture was also carried out using a liquid medium. That is to say, the cells were inoculated on a 60-mm dish (100 cells/4 mL of medium/dish), and on the 8^{th} day after the inoculation, the cells were fixed with methanol and were then stained with a Giemsa solution. Thereafter, the number of colonies per dish was counted. The number of soft agar colonies per number of surviving cells was obtained based on the obtained number of colonies. The obtained value was defmed as a soft agar colony formation rate.

**Table 1 Soft agar colony formation rate of Bhas preT cells**

| | Adhesion culture | | Soft agar culture | | |
|---|---|---|---|---|---|
| Cells | Number of cells inoculated (cells/dish) | Number of colonies (colonies/dish) | Number of cells inoculated (cells/dish) | Number of colonies (colonies/dish) | Colony formation rate (%) |
| BALB/c 3T3 | 100 | 64.3 | 10000 | 0 | 0 |
| Bhas 42 | 100 | 48.3 | 10000 | 30.3 | 0.6 |
| Bhas preT-1 | 100 | 54.0 | 1000 | 98.7 | 18.3 |
| Bhas preT-2 | 100 | 52.7 | 1000 | 135.7 | 25.8 |
| Bhas preT-3 | 100 | 58.3 | 1000 | 64.0 | 11.0 |
| Bhas preT-4 | 100 | 51.0 | 1000 | 24.7 | 4.8 |
| Bhas TPA-1 | 100 | 32.3 | 100 | 33.3 | 103.1 |

| | | | | | |
|---|---|---|---|---|---|
| *: (Number of colonies in soft agar medium / number of cells inoculated in soft agar medium) / (number of colonies in liquid medium / number of cells inoculated in liquid medium) x 100 | | | | | |

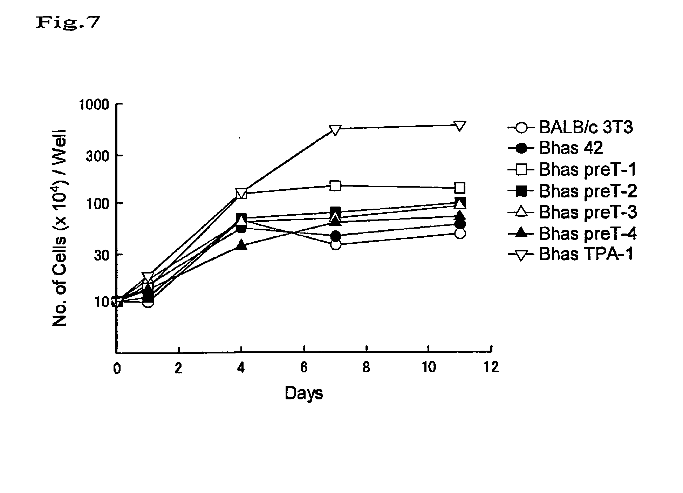

Moreover, various types of cells were each inoculated on a 6-well plate (10⁵ cells/2 mL of medium/well), and the cells were removed with trypsin over time, so that the number of cells per well was obtained. Using the obtained values, a growth curve was prepared, and the cell densities of various types of cells were compared with one another. Various types of Bhas preT cells were compared with BALB/c 3T3 cells, Bhas 42 cells, and completely transformed cells (Bhas TPA-1 cells). As a result, in terms of soft agar colony formation rate, all types of Bhas preT cells show the following results: BALB/c 3T3 cells < Bhas 42 cells < Bhas preT cells < Bhas T cells (Table 1). In terms of cell density as well, the following results were obtained: BALB/c 3T3 cells < Bhas 42 cells < Bhas preT cells < Bhas T cells (Figure 7). That is, it was found that the use of the present invention enables the selection of, not only transformed cells, but also pre-transformed cells.

### Example 5:

Whether or not human transformed cells can also be selected by treatment with hydrogen peroxide was analyzed. Since a quantitative transformation test using normal human cells has not yet existed to date, Hu-MI cells (human breast epithelial cells, which had acquired immortalization potential as a result of the introduction of an SV40 gene but did not exhibit tumorigenicity in nude mice, and which maintained a monolayer state) were considered to be normal cells. An experimental system was prepared, such that a large number of Hu-MI cells and a small number of human cancer cells (A549 cells: lung cancer; FLC-5 cells: liver cancer; HCT 116 cells: colon cancer; and MKN28 cells: stomach cancer) were simultaneously inoculated and such that they were co-cultured. After the cells had been cultured for a while, multilayered human cancer cells were formed as colonies in monolayered Hu-MI cells.

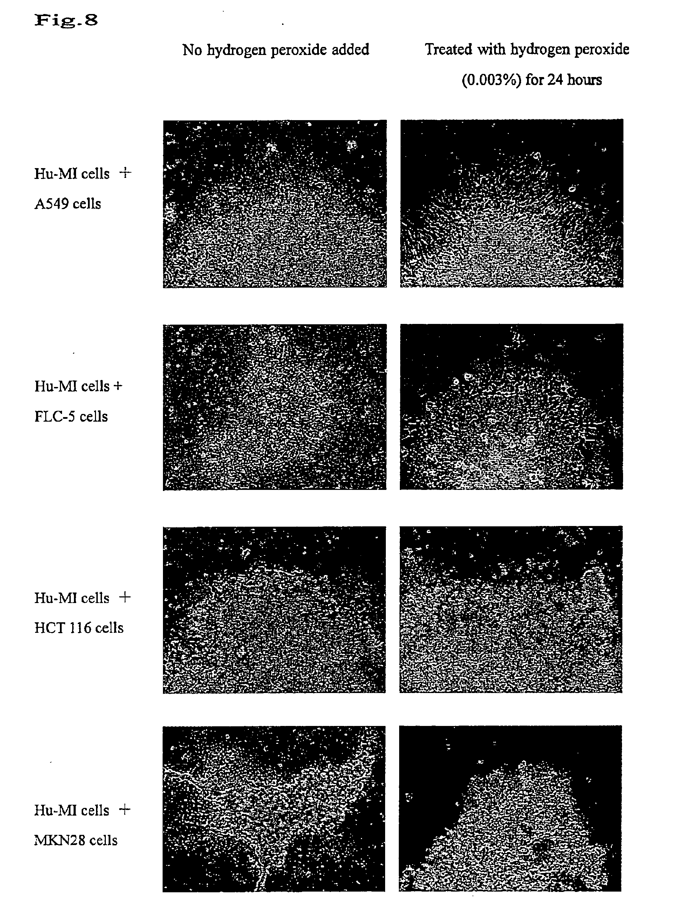

The Hu-MI cells (10⁴ cells/well) and various types of cancer cells (500 cells/well for FLC-5 cells; and 100 cells/well for other cells) were each inoculated on a 6-well plate (2 mL of medium/well). On the 14^{th} day after the inoculation, 0.2 mL of medium containing hydrogen peroxide (0.033%: 11 times higher than the final concentration that was 0.003%) was added thereto, and the cells were then observed 24 hours after the addition of hydrogen peroxide. As a medium, MEM + 10% fetal bovine serum was used. As a result, Hu-MI cells died and were thereby removed. In contrast, it was confirmed that all the human cancer cells remained in the state of colonies and survived (Figure 8). Not just the transformed mouse cells, but all the different types of human cancer cells could be selected using hydrogen peroxide. This result suggested that the present invention can be applied to any mammalian cells.

### Example 6:

Whether or not transformed cells can be selected using active oxygens other than hydrogen peroxide was analyzed. Since such active oxygens other than hydrogen peroxide are extremely unstable and have a short life time, they cannot be used as reagents. Accordingly, benzo(a)pyrene (BP) for generating superoxide anion radical as a result of light irradiation was utilized. Transformed foci were induced in Bhas 42 cells (by the method of Example 1), and the medium was then exchanged with a phosphate buffered saline (2 mL/well). Thereafter, 2 µL of a BP solution (20 µg/mL: 1,000 times higher than the final concentration that was 20 ng/mL) was added thereto. Using a sunlight simulator, light was applied to the cells (300 to 800 nm; UVA irradiation intensity: 5.6 mW/cm²; irradiation time: 6 minutes; irradiation dose: 2 J/cm²), and the medium was then exchanged. Twenty-four hours later, the resultant cells were observed.

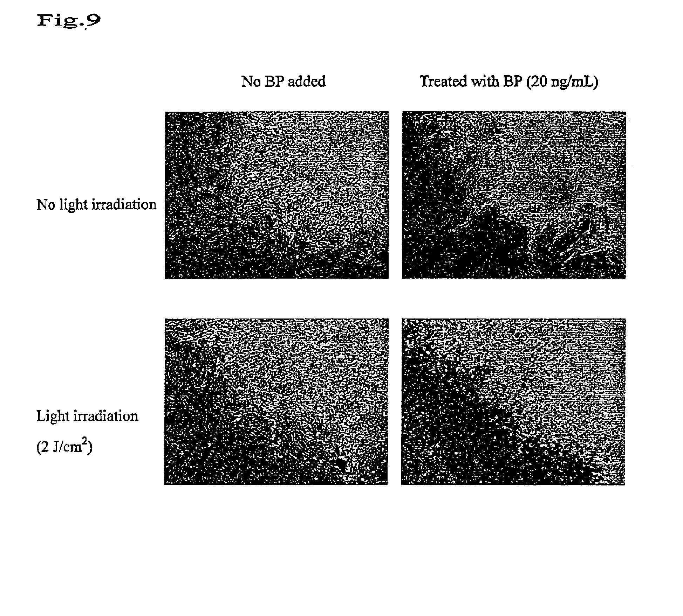

As a result, the selection of the transformed cells was observed only in the case of a BP treatment + light irradiation group, and no change was observed in other groups (Figure 9). When a BP treatment was combined with light irradiation, the same results as in the case of the addition of hydrogen peroxide were obtained. This results suggested that the transformed cells can be selected using any type of active oxygen.

### Example 7:

Whether or not the transformed cells can be selected even *in vivo* was analyzed. The following experiment was carried out using rasH2 mice (human c-Ha-ras gene-introduced mice; female; 6-week-old). N-ethyl-N-nitrosourea used as an initiator was intraperitoneally administered to each mouse (120 mg/kg/10 mL of physiological saline). From 1 week after the administration of N-ethyl-N-nirosourea, butylhydroxytoluene used as a promoter was orally administered once a week, repeatedly for 7 weeks (400 mg/kg/10mL of corn oil). Thereafter, the mouse was further bred for 2 weeks. Thereafter, tumor formed in the lung was excised together with the peripheral tissues, it was then sliced, and it was then added to a hydrogen peroxide-containing medium (final concentration: 0.015%). The medium was left at rest in a CO₂ incubator (5% CO₂, 37°C) for 24 hours, and the sample was then stained with a trypan blue solution (prepared by adding-0.002% trypan blue to phosphate buffered saline) for 10 minutes. It is to be noted that trypan blue is not incorporated into living cells but is incorporated into dead cells, and that it stains the cells blue.

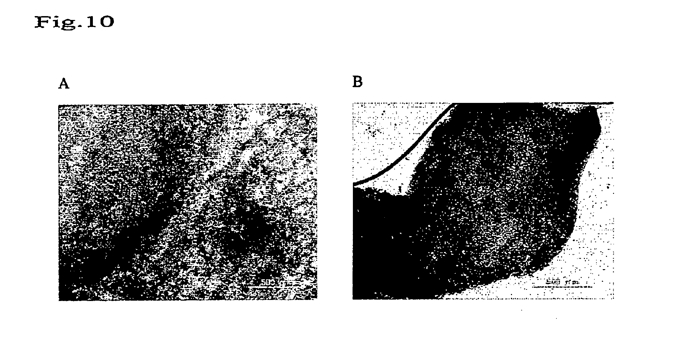

The presence of the tumor had been clearly observed at the time when the mouse had been excised. Thus, when its section was observed under a microscope, a tumor portion could be morphologically distinguished from a non-tumor portion. In a group that had not been treated with hydrogen peroxide, both a tumor portion and a non-tumor portion were not stained. In a group treated with hydrogen peroxide, only the non-tumor portion was stained blue (Figure 10). From the histological viewpoint, such tumor portion was constituted with tumor cells. Thus, it became clear that tumor existing *in vivo* can also be selected using hydrogen peroxide. Therefore, it was suggested that the present invention can be applied not only to the cultured cells, but also to cells existing in an individual mammal.

## Claims

1. An *in vitro* method for selecting transformed mammalian cells, which comprises treating a cell mixture comprising transformed mammalian cells and non-transformed mammalian cells with a solution containing active oxygen, so as to allow the transformed mammalian cells to selectively survive, wherein transformed mammalian cells include pre-transformed cells.

2. The method for selecting transformed mammalian cells according to claim 1, wherein the solution containing active oxygen is a solution containing hydrogen peroxide, single oxygen, superoxide anion radical, or hydroxyl radical.

3. The method for selecting transformed mammalian cells according to claim 1 or 2, wherein the transformed mammalian cells are pre-transformed cells.

4. The method for selecting transformed mammalian cells according to any of claims 1 to 3, wherein the transformed mammalian cells are cultured cells.

5. The method for selecting transformed mammalian cells according to claim 4, wherein the cultured transformed mammalian cells are Bhas 42 cells, BALB/c 3T3 cells, or human cancer cells.

6. The method for selecting transformed mammalian cells according to any of claims 1 to 5, wherein the transformed mammalian cells are cancer cells or cells that are in a precancerous state.

## Patentansprüche

1. Ein *in-vitro* Verfahren zur Selektion transformierter Säugetierzellen, das die Behandlung einer Zellmischung enthaltend transformierte Säugetierzellen und nicht transformierte Säugetierzellen mit einer Lösung enthaltend Aktivsauerstoff umfasst, um die transformierten Säugetierzellen selektiv überleben zu lassen, wobei die transformierten Säugetierzellen Zellen im Vortransformationsstadium einschließen.

2. Das Verfahren zur Selektion transformierter Säugetierzellen gemäß Anspruch 1, wobei die Lösung enthaltend Aktivsauerstoff eine Lösung ist, die Wasserstoffperoxid, Singulett-Sauerstoff, Hyperoxid-Radikal-Anion oder Hydroxylradikal enthält.

3. Das Verfahren zur Selektion transformierter Säugetierzellen gemäß Anspruch 1 oder 2, wobei die transformierten Zellen sich im Vortransformationsstadium befinden.

4. Das Verfahren zur Selektion transformierter Säugetierzellen gemäß irgendeinem der Ansprüche 1 bis 3, wobei die transformierten Säugetierzellen kultivierte Zellen sind.

5. Das Verfahren zur Selektion transformierter Säugetierzellen gemäß Anspruch 4, wobei die kultivierten transformierten Säugetierzellen Bhas42-Zellen, BALB/c 3T3-Zellen oder menschliche Krebszellen sind.

6. Das Verfahren zur Selektion transformierter Säugetierzellen gemäß irgendeinem der Ansprüche 1 bis 5, wobei die transformierten Säugetierzellen Krebszellen oder Zellen sind, die sich in einem Vorkrebsstadium befinden.

## Revendications

1. Procédé *in vitro* permettant de sélectionner des cellules mammaliennes transformées, qui comprend le traitement d'un mélange cellulaire comprenant des cellules mammaliennes transformées et des cellules mammaliennes non transformées avec une solution contenant de l'oxygène actif, de manière à permettre aux cellules mammaliennes transformées de survivre sélectivement, où les cellules mammaliennes transformées comprennent des cellules prétransformées.

2. Procédé pour sélectionner des cellules mammaliennes transformées selon la revendication 1, dans lequel la solution contenant de l'oxygène actif est une solution contenant du peroxyde d'hydrogène, de l'oxygène simple, le radical de l'anion superoxyde ou le radical hydroxyle.

3. Procédé pour sélectionner des cellules mammaliennes transformées selon la revendication 1 ou 2, dans lequel les cellules mammaliennes transformées sont des cellules prétransformées.

4. Procédé pour sélectionner des cellules mammaliennes transformées selon l'une quelconque des revendications 1 à 3, dans lequel les cellules mammaliennes transformées sont des cellules cultivées.

5. Procédé pour sélectionner des cellules mammaliennes transformées selon la revendication 4, dans lequel les cellules mammaliennes transformées cultivées sont des cellules Bhas 42, des cellules 3T3 de BALC/c ou des cellules cancéreuses humaines.

6. Procédé pour sélectionner des cellules mammaliennes transformées selon l'une quelconque des revendications 1 à 5, dans lequel les cellules mammaliennes transformées sont des cellules cancéreuses ou des cellules qui sont dans un état précancéreux.
